(19) Europäisches Patentamt
European Patent Office
Office européen des brevets

(11) Veröffentlichungsnummer: **0 128 530**
**B1**

(12) # EUROPÄISCHE PATENTSCHRIFT

(45) Veröffentlichungstag der Patentschrift: **27.04.88**

(51) Int. Cl.⁴: **C 07 D 237/14,** A 01 N 43/58

(21) Anmeldenummer: **84106490.1**

(22) Anmeldetag: **06.06.84**

(54) **Substituierte 4,5-Dimethoxypyridazone, Verfahren zu ihrer Herstellung, diese enthaltende Herbizide und ihre Verwendung zur Bekämpfung unerwünschten Pflanzenwuchses.**

(30) Priorität: **10.06.83 DE 3321007**

(43) Veröffentlichungstag der Anmeldung: **19.12.84 Patentblatt 84/51**

(45) Bekanntmachung des Hinweises auf die Patenterteilung: **27.04.88 Patentblatt 88/17**

(84) Benannte Vertragsstaaten: **AT BE CH DE FR GB IT LI NL**

(56) Entgegenhaltungen:
**EP-A-0 037 925**
**EP-A-0 085 785**
**DE-A-3 123 715**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Parg, Adolf, Dr., Paray- le- Monial-Strasse 8, D-6702 Bad Duerkheim (DE)**
Erfinder: **Hamprecht, Gerhard, Dr., Rote- Turm-Strasse 28, D-6940 Weinheim (DE)**
Erfinder: **Sauter, Hubert, Dr., Neckarpromenade, D-6800 Mannheim (DE)**
Erfinder: **Wuerzer, Bruno, Dr. Dipl.- Landwirt, Ruedigerstrasse 13, D-6701 Otterstadt (DE)**

**EP 0 128 530 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft substituierte 4,5-Dimethoxypyridazone, Verfahren zu ihrer Herstellung, Herbizide, die diese Pyridazone als Wirkstoffe enthalten, sowie Verfahren zur Bekämpfung unerwünschten Pflanzenwuchses mit diesen Wirkstoffen bzw. Herbiziden.

Es ist bekannt, daß 1-Phenyl-4,5-dimethoxy-pyridazon-(6) eine breite herbizide Wirkung hat und als Totalherbizid verwendet werden kann (US-PS-3 326 660). Der Wirkstoff besitzt pflanzenschädigende Eigenschaften sowohl bei Anwendung vor dem Anpflanzen der Pflanzen als auch bei Behandlung der Blätter. Außerdem sind 4,5-disubstituierte 1-(m-trifluormethyl-phenyl)-substituierte 6-Pyridazone sowie 4,5-disubstituierte 1-phenoxyphenylsubstituierte 6-Pyridazone mit ähnlichen herbiziden Eigenschaften bekannt (US-PS-3 697 522; US-PS-4 360 672).

Es wurde gefunden, daß substituierte 4,5-Dimethoxypyridazone der Formel I

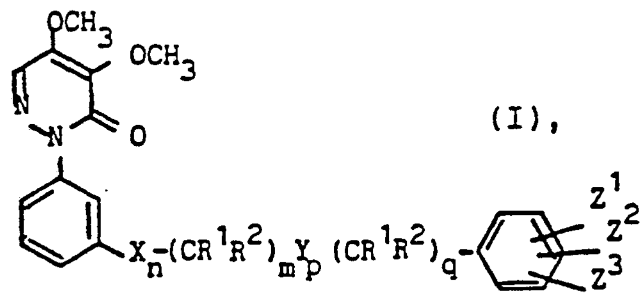

(I),

in der

X und Y jeweils unabhängig voneinander Sauerstoff, Schwefel, eine Sulfinylgruppe oder eine Sulfogruppe,

$R^1$ und $R^2$ jeweils unabhängig voneinander Wasserstoff oder $C_1$-$C_4$-Alkyl,

$Z^1$, $Z^2$ und $Z^3$ jeweils unabhängig voneinander Wasserstoff, Halogen, Nitro, Cyano, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylmercapto, $C_1$-$C_4$-Halogenalkylmercapto, $C_1$-$C_4$-Alkylsulfinyl oder $C_1$-$C_4$-Alkylsulfonyl bedeuten und

n und p für die Zahlen 0 oder 1,

m und q für eine Zahl von 0 bis 8 stehen, mit der Maßgabe, daß m und q nicht gleichzeitig für 0 stehen

eine gute herbizide Aktivität besitzen und dabei eine überraschende Verträglichkeit für Kulturpflanzen bzw. eine selektive herbizide Wirksamkeit bei Anwendung im Kulturpflanzenbau aufweisen. Die Substituenten in der Formel 1 können folgende Bedeutungen haben:

$R^1$ und $R^2$ können jeweils unabhängig voneinander Wasserstoff oder $C_1$-$C_4$-Alkyl, wie Methyl, Ethyl, n-Propyl, i-Propyl, n-Butyl oder tert.-Butyl bedeuten;

$Z^1$, $Z^2$ und $Z^3$ können jeweils unabhängig voneinander Wasserstoff, Halogen, wie Fluor, Chlor, Brom, Nitro, Cyano, $C_1$-$C_4$-Alkyl, wie Methyl, Ethyl, n-Propyl, i-Propyl, n-Butyl, tert.-Butyl, $C_1$-$C_4$-Halogenalkyl, wie Trifluormethyl, Difluormethyl, Fluormethyl, Trichlormethyl, Dichlormethyl, Chlormethyl, Difluorchlormethyl, 1-Chlorethyl, 2-Chlorethyl, 1-Fluorethyl, 2-Fluorethyl, 2,2,2-Trichlorethyl, 2,2,2-Trifluorethyl, 1,1,2,2-Tetrafluorethyl, 1,1,2-Trifluor-2-chlorethyl, 1,1,2,2,2-Pentafluorethyl, $C_1$-$C_4$-Alkoxy, wie Methoxy, Ethoxy, n-Propyloxy, i-Propyloxy, tert.-Butyloxy, $C_1$-$C_4$-Halogenalkoxy, wie Trichlormethoxy, Trifluormethoxy, 1-Chlorethoxy, 2-Chlorethoxy, 1-Fluorethoxy, 2-Fluorethoxy, 2,2,2-Trichlorethoxy, 2,2,2-Trifluorethoxy, 1,1,2,2-Tetrafluorethoxy, 1,1,2,2,2-Pentafluorethoxy, $C_1$-$C_4$-Alkylmercapto, wie Methylmercapto, Ethylmercapto, $C_1$-$C_4$-Halogenalkylmercapto, wie Trichlormethylmercapto, Trifluormethylmercapto, $C_1$-$C_4$-Alkylsulfinyl, wie Methylsulfinyl, Ethylsulfinyl, oder $C_1$-$C_4$ Alkylsulfonyl, wie Methylsulfonyl oder Ethylsulfonyl, bedeuten.

Bevorzugte Verbindungen der Formel I sind solche, bei denen

X und Y jeweils unabhängig voneinander Sauerstoff oder Schwefel,

$R^1$ und $R^2$ jeweils unabhängig voneinander Wasserstoff oder $C_1$-$C_4$-Alkyl,

$Z^1$, $Z^2$ und $Z^3$ jeweils unabhängig voneinander Wasserstoff, Halogen, $C_1$-$C_4$-Alkyl, $C_1$-$C_4$-Halogenalkyl, $C_1$-$C_4$-Alkoxy oder $C_1$-$C_4$-Halogenalkoxy bedeuten und

n und p für die Zahlen 0 oder 1,

m und q für eine Zahl von 0 bis 8 stehen, mit der Maßgabe, daß m und q nicht gleichzeitig für 0 stehen.

Weiterhin bevorzugt sind Verbindungen der Formel I, bei denen

$R^1$ und $R^2$ Wasserstoff,

$Z^1$ Wasserstoff,

$Z^2$ und $Z^3$ Halogen,

n 0,

m 1,

p 1 und

q 0 bedeuten.

Man erhält die 4,5-Dimethoxypyridazone der Formel I durch Umsetzung eines 4,5-Dihalogenpyridazons der Formel IV

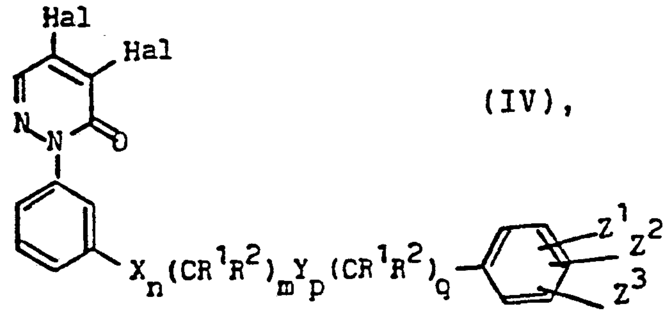

(IV),

in der X, Y, $R^1$, $R^2$, $Z^1$, $Z^2$, $Z^3$, n, m, p und q die obengenannten Bedeutungen haben und Hal Halogen, insbesondere Chlor oder Brom, bedeutet, mit ungefähr der stöchiometrischen Menge eines Alkalimethanolats in Gegenwart eines inerten organischen Lösungsmittels bei einer Temperatur von bis zu 100°C wobei man die Umsetzung drucklos oder unter Druck (1 bis 10 bar), kontinuierlich oder diskontinuierlich durchführen kann (Verfahren a).

Verwendet man 1-[3'-(4"-Chlorbenzyloxy)-phenyl]-4,5-dichlorpyridazon-(6) und Natriummethylat als Ausgangsmaterialien, so kann der Reaktionsablauf durch folgendes Formelschema wiedergegeben werden:

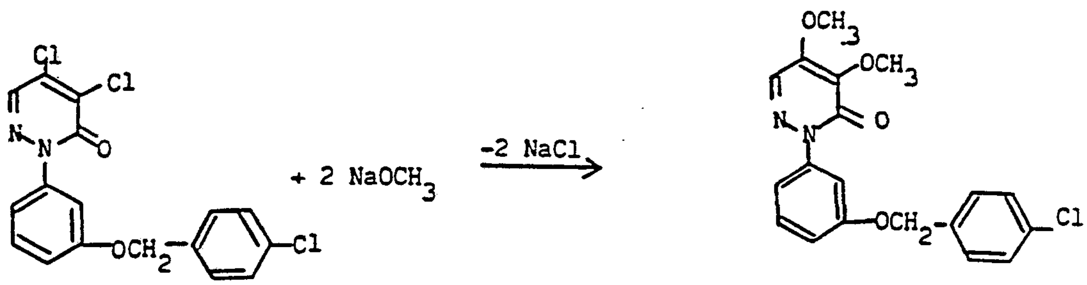


(IV) (I)

Zweckmäßigerweise wird das Dihalogenpyridazon IV zunächst in einem organischen Lösungsmittel, beispielsweise in Toluol, gelöst bzw. suspendiert und dann mit der entsprechenden Menge an Alkoholat umgesetzt. Die Reaktion verläuft bei Atmosphärendruck oder erhöhtem Druck, im allgemeinen innerhalb von 12 Stunden bei einer Reaktionstemperatur von bis zu 100°C, vorzugsweise bei einer Temperatur zwischen 40 und 80°C; die Umsetzung kann diskontinuierlich oder kontinuierlich durchgeführt werden. Das Reaktionsgemisch kann wie üblich aufgearbeitet werden: Fällt das Endprodukt fest an, so isoliert man es beispielsweise durch Absaugen des Niederschlags. Ist das Endprodukt dagegen im Lösungsmittel gelöst, wird dieses unter vermindertem Druck abdestilliert, der Rückstand wird mit Wasser verrührt und abgesaugt. Zur Reinigung kann das Produkt beispielsweise umkristallisiert oder chromatographiert werden.

Als Dihalogenpyridazone der Formel IV können Verbindungen verwendet werden, die beispielsweise durch Umsetzung eines Phenylhydrazins der Formel V

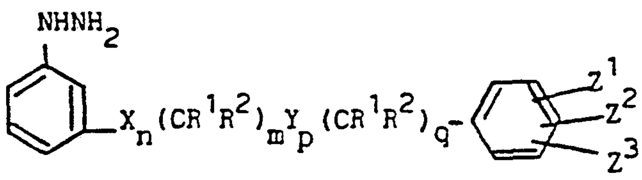

(V),

in der X, Y, $R^1$, $R^2$, $Z^1$, $Z^2$, $Z^3$, n, m, p und q die obengenannten Bedeutungen besitzen, mit einer 3-Formyl-2,3-dihalogen-acrylsäure der Formel VI

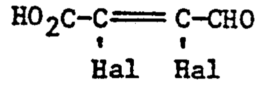

(VI),

in der Hal Chlor oder Brom bedeutet, und durch Cyclisierung des so erhaltenen Dihalogenacrylsäuresemicarbazons erhalten wurden.

Die Umsetzung zu dem entsprechenden Dihalogenacrylsäuresemicarbazon wird bei Raumtemperatur, z. B. in einer mineralsauren wäßrigen Lösung oder in einem wasserhaltigen oder wasserfreien, organischen Lösungsmittel, wie Ethanol, das nach Beendigung der Reaktion abgedampft wird, vorgenommen. Dieses Semicarbazon wird dann vorzugsweise ohne vorhergehende Isolierung, durch Kochen in Eisessig oder Essigsäureanhydrid oder durch Erwärmen in wäßriger Mineralsäure, z. B. Salzsäure, bei einer Temperatur von bis zu 100°C oder durch Rühren in konzentrierter Mineralsäure, z. B. Schwefelsäure bei Raumtemperatur zum Dihalogenpyridazon der Formel IV cyclisiert (DE-OS-1 695 840, DE-OS-2 526 643, DE-OS-1 545 595). Die Umsetzung kann diskontinuierlich oder kontinuierlich durchgeführt werden. Das Reaktionsprodukt läßt sich nach allgemein üblichen Methoden aufarbeiten.

Die 4,5-Dimethoxypyridazone der Formel I, in der X Sauerstoff oder Schwefel und n 1 bedeuten, können durch Umsetzung eines 4,5-Dimethoxypyridazons der Formel II

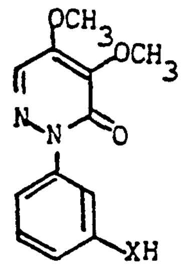

(II),

in der X Sauerstoff oder Schwefel bedeutet, mit ungefähr der stöchiometrischen Menge eines Halogenids der Formel III

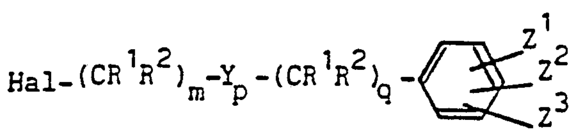

(III),

in der $R^1$, $R^2$, Y, $Z^1$, $Z^2$, $Z^3$, m, p und q die obengenannten Bedeutungen haben und Hal für Halogen, insbesondere Chlor oder Brom, steht, in Gegenwart eines inerten organische Lösungsmittels und gegebenenfalls eines Säureacceptors bei einer Temperatur von 0 bis 150°C hergestellt werden, wobei man die Umsetzung drucklos oder unter Druck, kontinuierlich oder diskontinuierlich durchführen kann (Verfahren b).

Verwendet man 1-(3'-Hydroxyphenyl)-4,5-dimethoxypyridazon-(6) und Benzylchlorid als Ausgangsmaterialien, so kann der Reaktionsablauf durch folgendes Formelschema wiedergegeben werden:

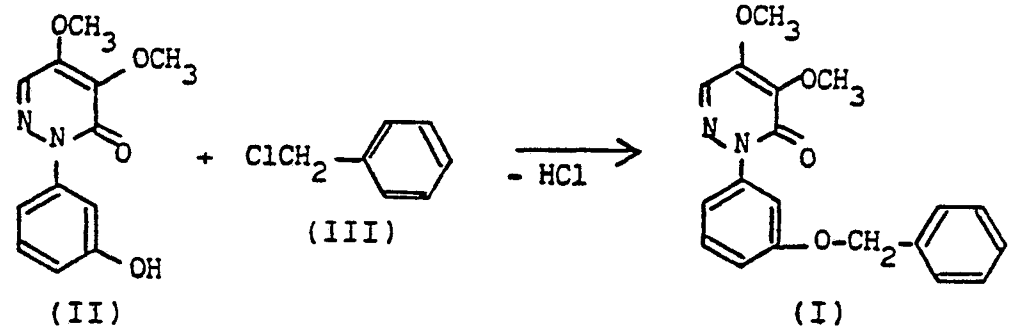

Die Ausgangsstoffe werden in ungefähr stöchiometrischem Verhältnis eingesetzt, d.h. in einem Unter- bzw. Überschuß von bis zu 10 % an Ausgangsstoff III, bezogen auf II. Gegebenenfalls kann ein Säureacceptor zur Vervollständigung der Reaktion zugesetzt werden. Ferner kann der bei der Umsetzung entstehende Halogenwasserstoff auch durch Einleiten eines inerten Gases, wie Stickstoff, ausgetrieben werden. Zweckmäßigerweise wird das Verfahren so durchgeführt, daß man eine Lösung des Benzylchlorids III bei 0 bis 30°C in einem inerten organischen Lösungsmittel, gegebenenfalls gleichzeitig mit der äquimolaren Menge eines Säureacceptors, zu einer Lösung bzw. Suspension des Ausgangsstoffes II in einem inerten organischen Lösungsmittel, beispielsweise Dimethylformamid, zulaufen läßt.

Zur Beendigung der Umsetzung rührt man 0,5 bis 48 Stunden, vorzugsweise 2 bis 12 Stunden, bei 30 bis 150°C nach. Das Reaktionsgemisch wird wie üblich aufgearbeitet. Fällt das Endprodukt fest an, so isoliert man es beispielsweise durch Absaugen des Niederschlags. Ist das Endprodukt dagegen im Lösungsmittel gelöst, wird dieses unter vermindertem Druck abdestilliert, der Rückstand wird dann mit Wasser verrührt und abgesaugt. Zur Reinigung kann das Produkt beispielsweise umkristallisiert oder chromatographiert werden.

Für beide Verfahren a) und b) können unter den jeweiligen Reaktionsbedingungen inerte organische Lösungsmittel verwendet werden. Geeignet sind beispielsweise Halogenkohlenwasserstoffe, insbesondere Chlorkohlenwasserstoffe, z. B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol; Ether, z. B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β'-Dichlordiethylether; Nitrokohlenwasserstoffe, wie Nitromethan, Nitroethan, Nitrobenzol, o-, m-, p-Chlornitrobenzol, o-Nitrotoluol; Nitrile, wie Acetonitril, Butyronitril, Isobutyronitril, Benzonitril, m-Chlorbenzonitril; aliphatische oder cycloaliphatische Kohlenwasserstoffe, z. B. Heptan, Pinan, Nonan, o-, m-, p-Cymol, Benzinfraktionen innerhalb eines Siedepunktintervalls von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Triethylpentan, Octan; Ester, z. B. Ethylacetat, Acetessigester, Isobutylacetat; Amide, z. B. Formamid, Methylformamid, Dimethylformamid; Ketone, z. B. Aceton, Methylethylketon; und entsprechende Gemische. Zweckmäßigerweise verwendet man das Lösungsmittel in einer Menge von 100 bis 2000 Gew. %, vorzugsweise von 200 bis 700 Gew.-%, bezogen auf die Ausgangsstoffe.

Als Säureacceptoren für Verfahren b) können alle üblichen Säurebindemittel verwendet werden. Hierzu gehören vorzugsweise tertiäre Amine, Erdalkaliverbindungen, Ammoniumverbindungen und Alkalimetallverbindungen sowie entsprechende Gemische. Es können aber auch Zinnverbindungen verwendet werden. Es kommen z. B. folgende basischen Verbindungen in Betracht: Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkhydrogencarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.-butylamin, Tribenzylamin, Tricyclohexylamin, Triamylamin, Diisopropylethylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, α-Picolin, γ-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N'-Dimethylcyclohexylamin, 2,6-Lutidin, 2,4-Lutidin, Trifurylamin, Triethylendiamin.

Die Ausgangsverbindungen werden nach literaturbekannten Methoden hergestellt. So erhält man die Phenylhydrazine der Formel V nach allgemein bekannten Methoden aus entsprechenden Anilinen durch

Diazotierung und Reduktion (Houben-Weyl, Methoden der organischen Chemie, Band 10/2, S. 180, Georg-Thieme-Verlag, Stuttgart, 1967). Die Umsetzung zum entsprechenden Pyrazon kann ohne Isolierung der Hydrazine geschehen, jedoch erhält man reinere Produkte, wenn man zuvor die Phenylhydrazine als Hydrochloride isoliert.

Die Aniline wiederum sind zum Teil literaturbekannt (DE-OS-2 846 723) bzw. können nach allgemein literaturbekannten Methoden hergestellt werden.

Die Oxidation von Thioethern zu entsprechenden Sulfoxiden bzw. Sulfonen ist allgemein bekannt und kann in an sich üblicher Weise durchgeführt werden.

Die folgenden Beispiele erläutern die Herstellung der Verbindungen der Formel I. Gewichtsteile verhalten sich zu Volumenteilen wie kg zu l.

**Beispiel 1**

a) Eine Suspension von 49,8 Gewichtsteilen 3-Phenoxymethylanilin in 400 Volumenteilen Eisessig und 70 Volumenteilen konz. Salzsäure wird mit einer Lösung von 17,25 Gewichtsteilen Natriumnitrit in 45 Volumenteilen Wasser bei 10 bis 20°C umgesetzt. Dann setzt man eine Lösung von 118 Gewichtsteilen $SnCl_2 \cdot 2H_2O$ in 8C Volumenteilen konz. Salzsäure zu, kühlt auf 0°C ab und isoliert das entsprechende Hydrazinhydrochlorid durch Absaugen.

b) Eine Suspension von 43,2 Gewichtsteilen des so isolierten Hydrazinhydrochlorids und 33,4 Gewichtsteilen Mucochlorsäure in 200 Volumenteilen Essigsäure wird 10 Minuten auf Siedetemperatur erhitzt, anschließend wird die Reaktionsmischung abgekühlt und in 1 000 Volumenteile Wasser eingerührt. Der Niederschlag wird dann abgesaugt und aus Ethanol umkristallisiert. Man erhält 53 Gewichtsteile 1-(3'-Phenoxymethyl-phenyl)-4,5-dichlorpyridazon-(6) vom Schmelzpunkt 92 bis 94°C.

c) Eine Suspension von 15 Gewichtsteilen 1-(3'-Phenoxymethyl-phenyl)-4,5-dichlorpyridazon-(6), 2,3 Gewichtsteilen Natriummethylat und 0,1 Gewichtsteilen N-Methylpyrrolidon in 150 Volumenteilen absolutem Toluol wird zwei Stunden unter Rühren bei 60°C gehalten. Die entstandene Lösung wird mit 200 Volumenteilen Ether verdünnt, zweimal mit je 100 Volumenteilen Wasser behandelt, getrocknet, filtriert und unter vermindertem Druck eingeengt. Der ölige Rückstand wird mit Diisoproylether verrieben. Man erhält durch Absaugen 13 Gewichtsteile (90 % d. Th.) 1-(3'-Phenoxymethyl-phenyl)-4,5-dimethoxypyridazon-(6) vom Schmelzpunkt 82 bis 85°C (Verbindung Nr. 1).

Nach der Vorschrift des vorstehenden Beispiels können die in der folgenden Tabelle 1 angeführten Verbindungen der Formel I hergestellt werden:

**Tabelle:**

| Verbindung Nr. | X | $(CR^1R^2)_m$ | Y | $(CR^1R^2)_q$ | 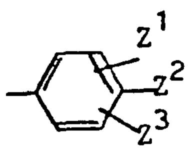 | Fp[°C]$n^{25}_D$/Wellenlänge einer Bande im IR-Spektrum |
|---|---|---|---|---|---|---|
| 2 | - | $CH_2$ | O | - | 4-Fluorphenyl | 95 - 99 |
| 3 | - | $CH_2$ | O | - | 3-Fluorphenyl | 108 - 112 |
| 4 | - | $CH_2$ | O | - | 2-Fluorphenyl | |
| 5 | - | $CH_2$ | O | - | 4-Chlorphenyl | 77 - 80 |
| 6 | - | $CH_2$ | O | - | 3-Chlorphenyl | |
| 7 | - | $CH_2$ | O | - | 2-Chlorphenyl | |
| 8 | - | $CH_2$ | O | - | 4-Bromphenyl | 1,6197 |
| 9 | - | $CH_2$ | O | - | 3-Bromphenyl | |
| 10 | - | $CH_2$ | O | - | 2-Bromphenyl | |
| 11 | - | $CH_2$ | O | - | 2,4-Difluorphenyl | |
| 12 | - | $CH_2$ | O | - | 3,4-Difluorphenyl | |
| 13 | - | $CH_2$ | O | - | 2,4-Dichlorphenyl | 89 - 93 |
| 14 | - | $CH_2$ | O | - | 3,4-Dichlorphenyl | 91 - 95 |
| 15 | - | $CH_2$ | O | - | 3,5-Dichlorphenyl | |
| 16 | - | $CH_2$ | O | - | 2,4-Dibromphenyl | |
| 17 | - | $CH_2$ | O | - | 3,4-Dibromphenyl | |
| 18 | - | $CH_2$ | O | - | 2-Chlor-4-fluorphenyl | |
| 19 | - | $CH_2$ | O | - | 2-Brom-4-fluorphenyl | |
| 20 | - | $CH_2$ | O | - | 2-Chlor-4-bromphenyl | |
| 21 | - | $CH_2$ | O | - | 2,4,6-Trichlorphenyl | |
| 22 | - | $CH_2$ | O | - | 4-Nitrophenyl | |
| 23 | - | $CH_2$ | O | - | 4-Cyanophenyl | |
| 24 | - | $CH_2$ | O | - | 4-Methylphenyl | 83 - 86 |

| | | | | | | |
|---|---|---|---|---|---|---|
| 25 | - | $CH_2$ | O | - | 3-Methylphenyl | |
| 26 | - | $CH_2$ | O | - | 4-Ethylphenyl | |
| 27 | - | $CH_2$ | O | - | 3-Ethylphenyl | |
| 28 | - | $CH_2$ | O | - | 4-tert.-Butylphenyl | 50 - 55 |
| 29 | - | $CH_2$ | O | - | 3-tert.-Butylphenyl | |
| 30 | - | $CH_2$ | O | - | 2-Chlor-4-methylphenyl | |
| 31 | - | $CH_2$ | O | - | 3-Chlor-4-methylphenyl | |
| 32 | - | $CH_2$ | O | - | 4-Trifluormethylphenyl | |
| 33 | - | $CH_2$ | O | - | 3-Trifluormethylphenyl | 76 - 81 |
| 34 | - | $CH_2$ | O | - | 2-Trifluormethylphenyl | |
| 35 | - | $CH_2$ | O | - | 2-Chlor-4-trifluormethylphenyl | |
| 36 | - | $CH_2$ | O | - | 2,6-Dichlor-4-trifluormethylphenyl | |
| 37 | - | $CH_2$ | O | - | 4-Methoxyphenyl | |
| 38 | - | $CH_2$ | O | - | 3-Methoxyphenyl | |
| 39 | - | $CH_2$ | O | - | 4-Ethoxyphenyl | |
| 40 | - | $CH_2$ | O | - | 3-Ethoxyphenyl | |
| 41 | - | $CH_2$ | O | - | 2-Chlor-4-methoxyphenyl | |
| 42 | - | $CH_2$ | O | - | 4-Trifluormethoxyphenyl | |
| 43 | - | $CH_2$ | O | - | 3-Trifluormethoxyphenyl | |
| 44 | - | $CH_2$ | O | - | 3-Tetrafluorethoxyphenyl | |
| 45 | - | $CH_2$ | O | - | 3-Difluormethoxyphenyl | |
| 46 | - | $CH_2$ | O | - | 4-Methylmercaptophenyl | |
| 47 | - | $CH_2$ | O | - | 3-Methylmercaptophenyl | |
| 48 | - | $CH_2$ | O | - | 3-Trifluormethylmercaptophenyl | |
| 49 | - | $CH_2$ | O | - | 3-Methylsulfinylphenyl | |
| 50 | - | $CH_2$ | O | - | 3-Methylsulfonylphenyl | |
| 51 | - | $CH_2CH_2$ | O | - | Phenyl | |
| 52 | - | $CH_2CH_2$ | O | - | 4-Chlorphenyl | |
| 53 | - | $CH_2CH_2$ | O | - | 2,4-Dichlorphenyl | |
| 54 | - | $CH_2CH_2$ | O | - | 4-Trifluormethylphenyl | |
| 55 | - | $CH_2CH_2CH_2$ | O | - | Phenyl | |
| 56 | - | $CH_2CH_2CH_2$ | O | - | 4-Chlorphenyl | |
| 57 | - | $CH_2CH_2CH_2CH_2$ | O | - | Phenyl | |
| 58 | - | $CH_2CH_2CH_2CH_2$ | O | - | 4-Chlorphenyl | |
| 59 | - | $CH(CH_3)$ | O | - | Phenyl | |
| 60 | - | $CH_2CH(CH_3)CH_2$ | O | - | Phenyl | |
| 61 | - | $CH_2C(CH_3)_2$ | O | - | Phenyl | |
| 62 | O | $CH_2$ | - | - | Phenyl | |
| 63 | O | $CH_2$ | - | - | 4-Fluorphenyl | |
| 64 | O | $CH_2$ | - | - | 3-Fluorphenyl | 70 - 72 |
| 65 | O | $CH_2$ | - | - | 2-Fluorphenyl | |
| 66 | O | $CH_2$ | - | - | 4-Chlorphenyl | 40 - 48 |
| 67 | O | $CH_2$ | - | - | 3-Chlorphenyl | |
| 68 | O | $CH_2$ | - | - | 2-Chlorphenyl | |
| 69 | O | $CH_2$ | - | - | 4-Bromphenyl | |
| 70 | O | $CH_2$ | - | - | 3-Bromphenyl | |
| 71 | O | $CH_2$ | - | - | 2,4-Dichlorphenyl | |
| 72 | O | $CH_2$ | - | - | 2,6-Dichlorphenyl | 127 - 131 |
| 73 | O | $CH_2$ | - | - | 4-Methylphenyl | |
| 74 | O | $CH_2$ | - | - | 3-Methylphenyl | |
| 75 | O | $CH_2$ | - | - | 4-Ethylphenyl | |
| 76 | O | $CH_2$ | - | - | 3-Ethylphenyl | |
| 77 | O | $CH_2$ | - | - | 4-Trifluormethylphenyl | 55 - 60 |
| 78 | O | $CH_2$ | - | - | 3-Trifluormethylphenyl | |
| 79 | O | $CH_2$ | - | - | 2-Chlor-4-trifluormethylphenyl | |
| 80 | O | $CH_2$ | - | - | 4-Methoxyphenyl | |
| 81 | O | $CH_2$ | - | - | 3-Methoxyphenyl | |
| 82 | O | $CH_2$ | - | - | 4-Trifluormethoxyphenyl | |
| 83 | O | $CH_2$ | - | - | 3-Trifluormethoxyphenyl | |
| 84 | O | $CH_2$ | - | - | 4-Methylmercaptophenyl | |
| 85 | O | $CH_2$ | - | - | 3-Methylmercaptophenyl | |
| 86 | O | $CH_2$ | - | - | 3-Trifluormethylmercaptophenyl | |
| 87 | O | $CH_2CH_2$ | - | - | Phenyl | 80 - 83 |
| 88 | O | $CH_2CH_2$ | - | - | 4-Chlorphenyl | |
| 89 | O | $CH_2CH_2CH_2$ | - | - | Phenyl | 1,6023 |

| | | | | | | |
|---|---|---|---|---|---|---|
| 90 | O | $CH_2CH_2CH_2$ | - | - | 4-Chlorphenyl | |
| 91 | O | $CH(CH_3)$ | - | - | Phenyl | |
| 92 | O | $CH_2C(CH_3)_2$ | - | - | Phenyl | |
| 93 | O | $CH_2CH(CH_3)CH_2$ | - | - | Phenyl | |
| 94 | O | $CH_2$ | O | - | Phenyl | |
| 95 | O | $CH_2$ | O | - | 4-Chlorphenyl | 102 -105 |
| 96 | O | $CH_2CH_2$ | O | - | Phenyl | 94 - 98 |
| 97 | O | $CH_2CH_2$ | O | - | 4-Chlorphenyl | 118 - 21 |
| 98 | O | $CH_2CH_2CH_2$ | O | - | Phenyl | 55 - 60 |
| 99 | O | $CH2CH_2CH_2$ | O | - | 4-Chlorphenyl | |
| 100 | O | $CH_2CH_2CH_2CH_2$ | O | - | Phenyl | 1,5932 |
| 101 | O | $CH_2CH_2CH_2CH_2$ | O | - | 4-Chlorphenyl | 71 - 75 |
| 102 | - | $CH_2$ | O | $CH_2$ | Phenyl | 1,6013 |
| 103 | - | $CH_2$ | O | $CH_2$ | 4-Chlorphenyl | |
| 104 | - | $CH(CH_3)$ | O | $CH_2$ | Phenyl | 1,5854 |
| 105 | - | $CH_2$ | S | - | Phenyl | 85 - 90 |
| 106 | - | $CH_2$ | S | - | 4-Fluorphenyl | |
| 107 | - | $CH_2$ | S | - | 3-Fluorphenyl | |
| 108 | - | $CH_2$ | S | - | 4-Chlorphenyl | 70 - 74 |
| 109 | - | $CH_2$ | S | - | 3-Chlorphenyl | |
| 110 | - | $CH_2$ | S | - | 4-Bromphenyl | |
| 111 | - | $CH_2$ | S | - | 2,4-Dichlorphenyl | |
| 112 | - | $CH_2$ | S | - | 2,4,6-Trichlorphenyl | |
| 113 | - | $CH_2$ | S | - | 4-Cyanophenyl | |
| 114 | - | $CH_2$ | S | - | 4-Methylphenyl | |
| 115 | - | $CH_2$ | S | - | 3-Methylphenyl | |
| 116 | - | $CH_2$ | S | - | 2-Chlor-4-methylphenyl | |
| 117 | - | $CH_2$ | S | - | 4-Trifluormethylphenyl | |
| 118 | - | $CH_2$ | S | - | 3-Trifluormethylphenyl | |
| 119 | - | $CH_2$ | S | - | 2-Chlor-4-trifluormethylphenyl | |
| 120 | - | $CH_2$ | S | - | 4-Methoxyphenyl | |
| 121 | - | $CH_2$ | S | - | 3-Methoxyphenyl | |
| 122 | - | $CH_2$ | S | - | 3-Trifluormethoxyphenyl | |
| 123 | - | $CH_2CH_2$ | S | - | Phenyl | |
| 124 | - | $CH_2CH_2$ | S | - | 4-Chlorphenyl | |
| 125 | - | $CH_2CH_2CH_2$ | S | - | Phenyl | |
| 126 | S | $CH_2$ | - | - | Phenyl | 60 - 64 |
| 127 | S | $CH_2$ | - | - | 4-Fluorphenyl | 70 - 73 |
| 128 | S | $CH_2$ | - | - | 3-Fluorphenyl | 55 - 60 |
| 129 | S | $CH_2$ | - | - | 4-Chlorphenyl | 70 - 74 |
| 130 | S | $CH_2$ | - | - | 3-Chlorphenyl | 65 - 70 |
| 131 | S | $CH_2$ | - | - | 2-Chlorphenyl | |
| 132 | S | $CH_2$ | - | - | 4-Bromphenyl | |
| 133 | S | $CH_2$ | - | - | 3-Bromphenyl | |
| 134 | S | $CH_2$ | - | - | 2,4-Dichlorphenyl | |
| 135 | S | $CH_2$ | - | - | 2,6-Dichlorphenyl | 82 - 85 |
| 136 | S | $CH_2$ | - | - | 2-Cyanphenyl | 1,6371 |
| 137 | S | $CH_2$ | - | - | 4-Methylphenyl | 70 - 75 |
| 138 | S | $CH_2$ | - | - | 3-Methylphenyl | 1,6374 |
| 139 | S | $CH_2$ | - | - | 4-Trifluormethylphenyl | 50 - 53 |
| 140 | S | $CH_2$ | - | - | 3-Trifluormethylphenyl | |
| 141 | S | $CH_2$ | - | - | 2-Chlor-4-trifluormethylphenyl | |
| 142 | S | $CH_2$ | - | - | 4-Methoxyphenyl | |
| 143 | S | $CH_2$ | - | - | 3-Methoxyphenyl | |
| 144 | S | $CH_2$ | - | - | 4-Trifluormethoxyphenyl | |
| 145 | S | $CH_2$ | - | - | 3-Trifluormethoxyphenyl | |
| 146 | S | $CH_2CH_2$ | - | - | Phenyl | |
| 147 | S | $CH_2CH_2$ | - | - | 4-Chlorphenyl | |
| 148 | S | $CH_2CH_2CH_2$ | - | - | Phenyl | |
| 149 | S | $CH_2CH_2CH_2$ | - | - | 4-Chlorphenyl | |
| 150 | S | $CH_2CH_2CH_2CH_2$ | - | - | Phenyl | |
| 151 | S | $CH_2CH_2CH_2CH_2$ | - | - | 4-Chlorphenyl | |
| 152 | S | $CH(CH_3)_2$ | - | - | 4-Chlorphenyl | |
| 153 | S | $CH(CH_3)_2$ | - | - | Phenyl | |
| 154 | S | $CH_2CH(CH_3)CH_2$ | - | - | Phenyl | |

| | | | | | |
|---|---|---|---|---|---|
| 155 | S | $CH_2$ | O | - | Phenyl |
| 156 | S | $CH_2$ | O | - | 4-Chlorphenyl |
| 157 | S | $CH_2CH_2$ | O | - | Phenyl |
| 158 | S | $CH_2CH_2$ | O | - | 4-Chlorphenyl |
| 159 | S | $CH_2CH_2CH_2$ | O | - | Phenyl |
| 160 | S | $CH_2CH_2CH_2$ | O | - | 4-Chlorphenyl |
| 161 | S | $CH_2CH_2CH_2CH_2$ | O | - | Phenyl |
| 162 | S | $CH_2$ | S | - | Phenyl |
| 163 | S | $CH_2CH_2$ | S | - | Phenyl |
| 164 | S | $CH_2CH_2CH_2$ | S | - | Phenyl |
| 165 | O | $CH_2$ | S | - | Phenyl |
| 166 | O | $CH_2CH_2$ | S | - | Phenyl |
| 167 | O | $CH_2CH_2CH_2$ | S | - | Phenyl |
| 168 | - | $CH_2$ | SO | - | Phenyl |
| 169 | - | $CH_2$ | $SO_2$ | - | Phenyl |
| 170 | - | $CH_2$ | SO | - | 4-Chlorphenyl |
| 171 | - | $CH_2$ | $SO_2$ | - | 4-Chlorphenyl |
| 172 | SO | $CH_2$ | - | - | Phenyl |
| 173 | $SO_2$ | $CH_2$ | - | - | Phenyl |
| 174 | SO | $CH_2$ | - | - | 4-Fluorphenyl |
| 175 | $SO_2$ | $CH_2$ | - | - | 4-Fluorphenyl |
| 176 | SO | $CH_2$ | - | - | 3-Chlorphenyl |
| 177 | $SO_2$ | $CH_2$ | - | - | 3-Chlorphenyl |
| 178 | SO | $CH_2$ | - | - | 4-Trifluormethylphenyl |
| 179 | $SO_2$ | $CH_2$ | - | - | Phenyl |
| 180 | - | $CH_2$ | S | $CH_2$ | Phenyl |
| 181 | - | $CH_2$ | SO | $CH_2$ | Phenyl |
| 182 | - | $CH_2$ | SO | $CH_2$ | Phenyl |

Die 4,5-Dimethoxypyridazone der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.

B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

**Beispiele für Formulierungen sind:**

I. Man vermischt 90 Gewichtsteile der Verbindung Nr. 1 mit 10 Gewichtsteilen N-Methyl-alpha-pyrrolidon und erhält eine Lösung, die zur Anwendung in Form kleinster Tropfen geeignet ist.

II. 10 Gewichtsteile der Verbindung Nr. 3 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-monoethanolamid, 2 Gewichtsteilen Calciumsalz der Dodecyl-benzolsulfonsäure und 2 Gewichtsteilen des Anlagerungsproduktes von 40 Mol Ethylenoxid an 1 Mol Ricinusöl besteht.

III. 20 Gewichtsteile der Verbindung Nr. 5 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

IV. 20 Gewichtsteile der Verbindung Nr. 33 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 80 Gewichtsteile der Verbindung Nr. 126 werden mit 3 Gewichtsteilen des Natriumsalzes der Diisobutylnaphthalin-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gewichtsteile der Verbindung Nr. 130 werden mit 95 Gewichtsteilen feinteiligem Kaolin vermischt. Man erhält auf diese Weise ein Stäubemittel, das 5 Gewichtsprozent des Wirkstoffs enthält.

VII. 30 Gewichtsteile der Verbindung Nr. 108 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 28 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe bei Nachauflaufanwendung für gewisse Kulturpflanzen weniger verträglich, können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (postdirected, lay-by).

Die Aufwandmengen an Wirkstoff betragen je nach Jahreszeit, Zielpflanzen und Wachstumsstadium, 0,05 bis 3 kg/ha, vorzugsweise 0,1 bis 1 kg/ha.

Die herbizide Wirkung der 4,5-Dimethoxypyridazone der Formel I läßt sich durch Gewächshausversuche zeigen:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Bei dierser Applikationsmethode beträgt die Aufwandmenge 3,0 kg Wirkstoff/ha. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Es werden entweder direkt gesäte und in den gleichen Gefäßen aufgewachsene Pflanzen verwendet, oder aber solche, die erst als Keimpflanzen getrennt angezogen und einige Tage vor der Behandlung in die Versuchsgefäße verpflanzt werden. Die Aufwandmenge beträgt 0,25 bzw. 0,5 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:.

Abutilon theophrasti (Chinesischer Hanf), Amaranthus retroflexus (Zurückgekrümmter Fuchsschwanz), Arachis hypogaea (Erdnuß), Cassia tora, Centaurea cyanus (Kornblume), Chenopodium album (Weißer Gänsefuß), Datura stramonium (Gemeiner Stechapfel), Desmodium tortuosum, Euphorbia geniculata (Südamerik. Wolfsmilch), Galium aparine (Klettenlabkraut), Helianthus annuus (Sonnenblumen), Ipomoea spp. (Prunkwindearten), Lamium spp. (Taubnesselarten), Mercurialis annua (Einjähriges Bingelkraut), Polygonum persicaria (Flohknöterich), Sinapis alba (Weißer Senf), Triticum aestivum (Weizen).

Bei der Prüfung auf herbizide Eigenschaften zeigen beispielsweise die Verbindungen Nr. 1, 3, 28, 33, 108 und 126 bei Vorauflaufanwendung von 3,0 kg Wirkstoff/ha eine sehr gute Aktivität an Sinapis alba.

Im Nachauflaufverfahren haben beispielsweise die Verbindungen Nr. 5, 129, 130 und 137 bei einer Aufwandmenge von beispielsweise 0,25 kg Wirkstoff/ha eine beachtliche herbizide Wirkung gegen eine Reihe von breitblättrigen unerwünschten Pflanzen. Außerdem bekämpfen die Verbindungen Nr. 33 und 3 unerwünschte Pflanzen mit 0,25 kg Wirkstoff bzw. 0,5 kg/ha selektiv, wobei beispielsweise Kulturen, wie Erdnüsse und Weizen, gar nicht oder nur geringfügig geschädigt werden. Breitblättrige unerwünschte Pflanzen in Sonnenblumen werden beispielsweise mit einer Aufwandmenge von 0,125 bzw. 0,25 kg Wirkstoff/ha selektiv durch die Verbindungen 3, 14, 33 und 126 bekämpft.

In Anbetracht der Vielseitigkeit der Applikationsmethoden können die 4,5-Dimethoxypyridazone der Formel I noch in einer weiteren Zahl von Kulturpflanzen zur Beseitigung von unerwünschtem Pflanzenwuchs, vorzugsweise breitblättriger annueller Arten, eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceium, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |

| | |
|---|---|
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Tibes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die 4,5-Dimethoxypyridazone der Formel I bzw. die sie enthaltenden Mittel auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Triazinone, Uracile, Benzofuranderivate, Cyclohexan-1,3-dion-derivate und andere in Betracht.

Außerdem kann es von Nutzen sein, die erfindungsgemäßen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden. Es können auch nichtphytotoxische Öle und Ölkonzentrate zugesetzt werden.

**Patentansprüche**

1. Substituierte 4,5-Dimethoxypyridazone der Formel I

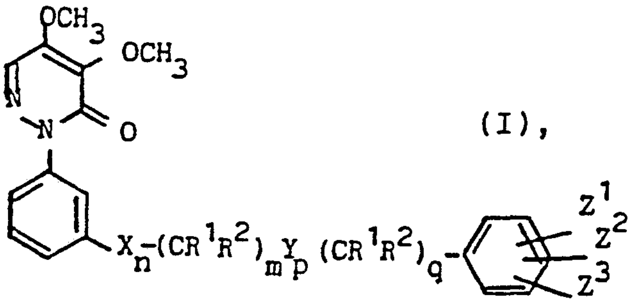

(I),

in der

X und Y jeweils unebhängig voneinander Sauerstoff, Schwefel, eine Sulfinylgruppe oder eine Sulfogruppe,

$R^1$ und $R^2$ jeweils unabhängig voneinander Wasserstoff oder $C_1$-$C_4$-Alkyl,

$Z^1$, $Z^2$ und $Z^3$ jeweils unabhängig voneinander Wasserstoff, Halogen, Nitro, Cyano, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylmercapto, $C_1$-$C_4$-Halogenalkylmercapto, $C_1$-$C_4$-Alkylsulfinyl oder $C_1$-$C_4$-Alkylsulfonyl bedeuten und

n und p für die Zahlen 0 oder 1,

m und q für eine Zahl von 0 bis 8 stehen, mit der Maßgabe, daß m und q nicht gleichzeitig für 0 stehen.

2. Substituierte 4,5-Dimethoxypyridazone der Formel I gemäß Anspruch 1, dadurch gekennzeichnet, daß

X und Y jeweils unabhängig voneinander Sauerstoff oder Schwefel,

$R^1$ und $R^2$ jeweils unabhängig voneinander Wasserstoff oder $C_1$-$C_4$-Alkyl,

$Z^1$, $Z^2$ und $Z^3$ jeweils unabhängig voneinander Wasserstoff, Halogen, $C_1$-$C_4$-Alkyl, $C_1$-$C_4$-Halogenalkyl, $C_1$-$C_4$-Alkoxy oder $C_1$-$C_4$-Halogenalkoxy bedeuten und

n und p für die Zahlen 0 oder 1,

m und q für eine Zahl von 0 bis 8 stehen, mit der Maßgabe, daß m und q nicht gleichzeitig für 0 stehen.

3. Substituierte 4,5-Dimethoxypyridazone der Formel I gemäß Anspruch 1, dadurch gekennzeichnet, daß

$R^1$ und $R^2$ Wasserstoff,

$Z^1$ Wasserstoff,

$Z^2$ und $Z^3$ Halogen,

n 0,

m 1,

p 1 und

q 0 bedeuten.

4. Verfahren zur Herstellung substituierter 4,5-Dimethoxypyridazone der Formel I gemäß Anspruch 1, dadurch gekennzeichnet, daß man ein Dihalogenpyridazon der Formel IV

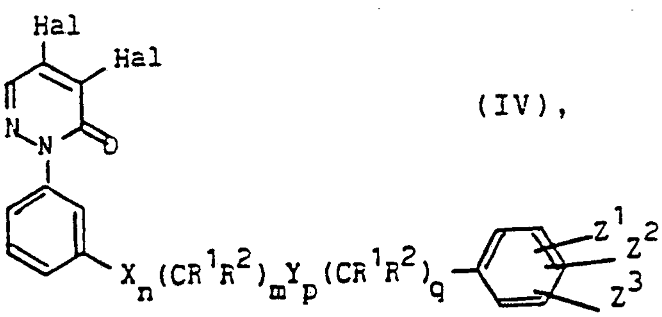

(IV),

in der X, Y, $R^1$, $R^2$, $Z^1$, $Z^2$, $Z^3$, n, m, p und q die im Anspruch 1 genannten Bedeutungen haben und Hal für Halogen steht, mit ungefähr der stöchiometrischen Menge eines Alkalimethanolats in Gegenwart eines inerten organischen Lösungsmittels bei einer Temperatur von bis zu 100°C umsetzt.

5. Verfahren zur Herstellung substituierter 4,5-Dimethoxypyridazone gemäß Anspruch 4, dadurch gekennzeichnet, daß man ein Dihalogenpyridazon der Formel IV verwendet, das durch Umsetzung eines Phenylhydrazins der Formel

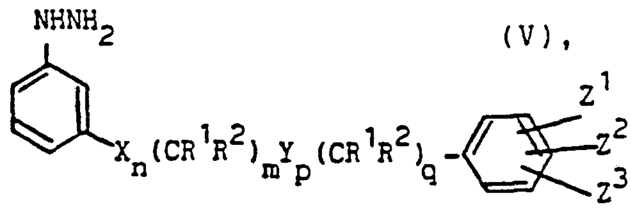

(V),

in der X, Y, $R^1$, $R^2$, $Z^1$, $Z^2$, $Z^3$, n, m, p und q die im Anspruch 1 genannten Bedeutungen haben, mit einer 3-Formyl-2,3-dihalogen-acrylsäure der Formel VI

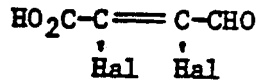

(VI),

in der Hal Chlor oder Brom bedeutet, zunächst bei Raumtemperatur in Gegenwart eines Lösungsmittels zum entsprechenden Dihalogenacrylsäuresemicarbazon und durch Cyclisierung dieses Semicarbazons hergestellt wird.

6. Verfahren zur Herstellung substituierter 4,5-Dimethoxypyridazone der Formel I gemäß Anspruch 1, wobei X Sauerstoff oder Schwefel und n 1 bedeuten, dadurch gekennzeichnet, daß man ein 4,5-Dimethoxypyridazon der Formel II

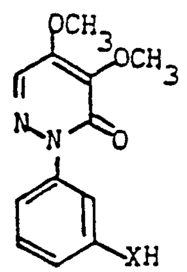

(II),

in der X Sauerstoff oder Schwefel bedeutet, mit ungefähr der stöchiometrischen Menge eines Alkylhalogenids der Formel III

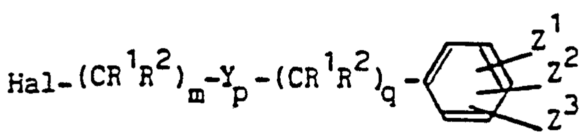

(III),

in der $R^1$, $R^2$, Y, $Z^1$, $Z^2$, $Z^3$, m, p und q die im Anspruch 1 genannten Bedeutungen haben und Hal für Halogen steht, in Gegenwart eines inerten organischen Lösungsmittels und gegebenenfalls eines Säureacceptors bei einer Temperatur von 0 bis 150°C umsetzt.

7. Herbizid, enthaltend ein 4,5-Dimethoxypyridazon der Formel I gemäß Anspruch 1.

8. Herbizid, enthaltend inerte Zusatzstoffe und ein 4,5-Dimethoxypyridazon der Formel I

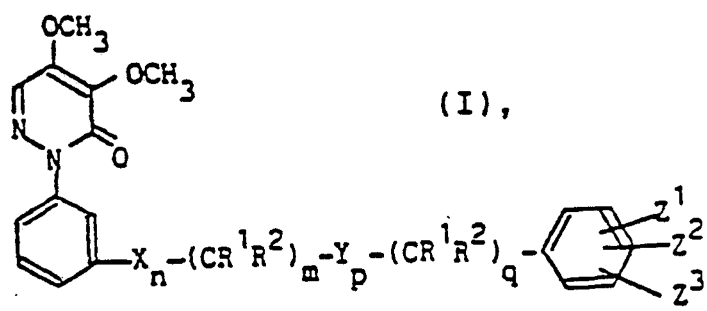

(I),

in der

X und Y jeweils unabhängig voneinander Sauerstoff, Schwefel, eine Sulfinylgruppe oder eine Sulfogruppe,

$R^1$ und $R^2$ jeweils unabhängig voneinander Wasserstoff oder $C_1$-$C_4$-Alkyl,

$Z^1$, $Z^2$ und $Z^3$ jeweils unabhängig voneinander Wasserstoff, Halogen, Nitro, Cyano, $C_1$-$C_4$-Alkyl, $C_1$-$C_4$-

Halogenalkyl, $C_1$-$C_4$-Alkoxy, $C_1$-$C_4$-Halogenalkoxy, $C_1$-$C_4$-Alkylmercapto, $C_1$-$C_4$-Halogenalkylmercapto, $C_1$-$C_4$-Alkylsulfinyl oder $C_1$-$C_4$-Alkylsulfonyl bedeuten und

n und p für die Zahlen 0 oder 1

m und q für eine Zahl von 0 bis 8 stehen, mit der Maßgabe, daß m und q nicht gleichzeitig für 0 stehen.

9. Herbizid, enthaltend inerte Zusatzstoffe und ein 4,5-Dimethoxypyridazon der Formel I gemäß Anspruch 1, wobei X und Y jeweils unabhängig voneinander Sauerstoff oder Schwefel,

$R^1$ und $R^2$ jeweils unabhängig voneinander Wasserstoff oder $C_1$-$C_4$-Alkyl,

$Z^1$, $Z^2$ und $Z^3$ jeweils unabhängig voneinander Wasserstoff, Halogen, $C_1$-$C_4$-Alkyl, $C_1$-$C_4$-Halogenalkyl, $C_1$-$C_4$-Alkoxy oder $C_1$-$C_4$-Halogenalkoxy bedeuten und

n und p für die Zahlen 0 oder 1

m und q für eine Zahl von 0 bis 8 stehen, mit der Maßgabe, daß m und q nicht gleichzeitig für 0 stehen.

10. Verfahren zur Bekämpfung unerwünschten Pflanzenwachstums, dadurch gekennzeichnet, daß man die unerwünschten Pflanzen oder die von unerwünschtem Pflanzenwachstum freizuhaltenden Flächen mit einer herbizid wirksamen Menge eines 4,5-Dimethoxypyridazons der Formel I gemäß Anspruch 1 behandelt.

## Claims

1. A substituted 4,5-dimethoxypyridazone of the formula I

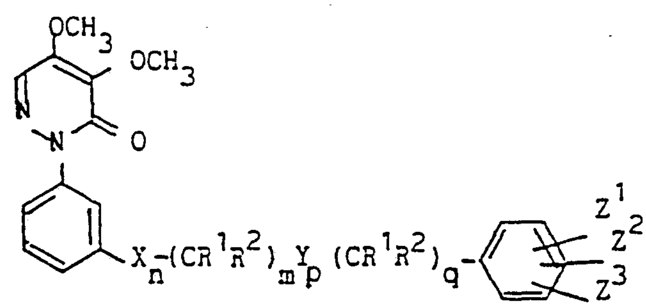

(I),

where

X and Y independently of one another are oxygen, sulfur, a sulfinyl group or a sulfo group,

$R^1$ and $R^2$ independently of one another are hydrogen or $C_1$-$C_4$-alkyl,

$Z^1$, $Z^2$ and $Z^3$ independently of one another are hydrogen, halogen, nitro, cyano, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylmercapto, $C_1$-$C_4$-haloalkylmercapto, $C_1$-$C_4$-alkylsulfinyl or $C_1$-$C_4$-alkylsulfonyl,

n and p are each 0 or 1, and

m and q are each 0 to 8, with the proviso that m and q do not each simultaneously denote 0.

2. A substituted 4,5-dimethoxypyridazone of the formula I as claimed in claim 1, where

X and Y independently of one another are oxygen or sulfur,

$R^1$ and $R^2$ independently of one another are hydrogen or $C_1$-$C_4$-alkyl,

$Z^1$, $Z^2$ and $Z^3$ independently of one another are hydrogen, halogen, $C_1$-$C_4$-alkyl, $C_1$-$C_4$-haloalkyl, $C_1$-$C_4$-alkoxy or $C_1$-$C_4$-haloalkoxy,

n and p are each 0 or 1, and

m and q are each 0 to 8, with the proviso that m and q do not each simultaneously denote 0.

3. A substituted 4,5-dimethoxypyridazone of the formula I as claimed in claim 1, where

$R^1$ and $R^2$ are each hydrogen,

$Z^1$ is hydrogen,

$Z^2$ and $Z^3$ are each halogen,

n is 0,

m is 1,

p is 1, and

q is 0.

4. A process for the preparation of a substituted 4,5-dimethoxypyridazone of the formula I as claimed in claim 1, wherein a dihalopyridazone of the formula IV

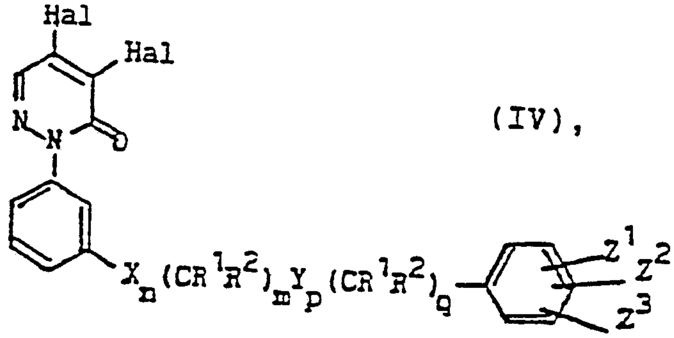

(IV),

where X, Y, $R^1$, $R^2$, $Z^1$, $Z^2$, $Z^3$, n, m, p and q have the meanings given in claim 1, and Hal is halogen, is reacted with about the stoichiometric amount of an alkali metal methylate in the presence of an inert organic solvent at a temperature of up to 100°C.

5. A process for the preparation of a substituted 4,5-dimethoxypyridazone, as claimed in claim 4, wherein a dihalopyridazone of the formula IV is used which is obtained by reacting a phenylhydrazine of the formula V

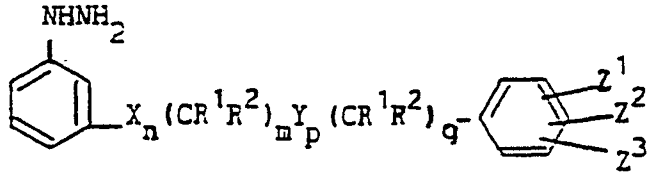

(V)

where X, Y, $R^1$, $R^2$, $Z^1$, $Z^2$, $Z^3$, n, m, p and q have the meanings given in claim 1, with a 3-formyl-2,3-dihaloacrylic acid of the formula VI

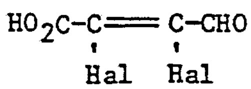

(VI),

where Hal is chlorine or bromine, at room temperature in the presence of a solvent to give the corresponding dihaloacrylic acid semicarbazone, and then subjecting this semicarbazone to a cyclization reaction.

6. A process for the preparation of a substitued 4,5-dimethoxypyridazone of the formula I as claimed in claim 1, where X is oxygen or sulfur, and n is 1, wherein a 4,5-dimethoxypyridazone of the formula II

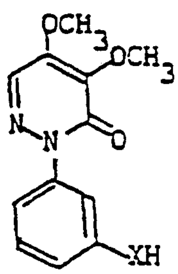

(II)

where X is oxygen or sulfur, is reacted with about the stoichiometric amount of an alkyl halide of the formula III

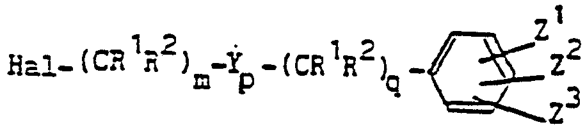

(III)

where $R^1$, $R^2$, Y, $Z^1$, $Z^2$, $Z^3$, m, p and q have the meanings given in claim 1, and Hal is halogen, in the presence of an inert organic solvent and in the presence or absence of an acid acceptor at from 0 to 150°C.

7. A herbicide containing a 4,5-dimethoxypyridazone of the formula I as claimed in claim 1.

8. A herbicide containing inert additives and a 4,5-dimethoxypyridazone of the formula I

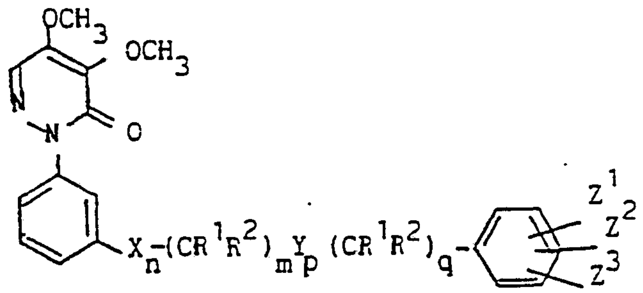

(I),

where

X and Y independently of one another are oxygen, sulfur, a sulfinyl group or a sulfo group,

$R^1$ and $R^2$ independently of one another are hydrogen or $C_1$-$C_4$-alkyl,

$Z^1$, $Z^2$ and $Z^3$ independently of one another are hydrogen, halogen, nitro, cyano, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylmercapto, $C_1$-$C_4$-haloalkylmercapto, $C_1$-$C_4$-alkylsulfinyl or $C_1$-$C_4$-alkylsulfonyl,

n and p are each 0 or 1, and

m and q are each 0 to 8, with the proviso that m and q do not each simultaneously denote 0.

9. A herbicide containing inert additives and a 4,5-dimethoxypyridazone of the formula I as claimed in claim 1, where

X and Y independently of one another are oxygen or sulfur,

$R^1$ and $R^2$ independently of one another are hydrogen or $C_1$-$C_4$-alkyl,

$Z^1$, $Z^2$ and $Z^3$ independently of one another are hydrogen, halogen, $C_1$-$C_4$-alkyl, $C_1$-$C_4$-haloalkyl, $C_1$-$C_4$-alkoxy or $C_1$-$C_4$-haloalkoxy,

n and p are each 0 or 1, and

m and q are each 0 to 8, with the proviso that m and q do not each simultaneously denote 0.

10. A process for combatting the growth of unwanted plants, wherein the unwanted plants or the areas to be kept free from unwanted plants are treated with a herbicidally effective amount of a 4,5-dimethoxypyridazone of the formula I as claimed in claim 1.

**Revendications**

1. Diméthoxy-4,5 pyridazones substituées de la formule I

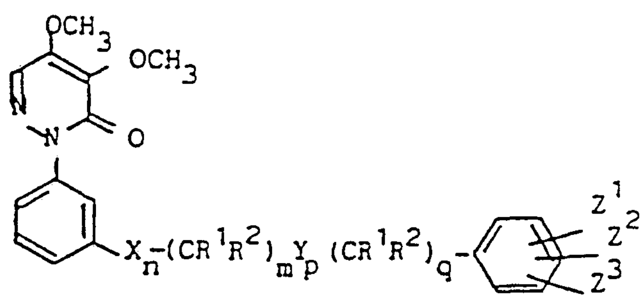

(I),

dans laquelle

X et Y peuvent désigner chacun, indépendamment l'un de l'autre, un atome d'oxygène ou de soufre ou un groupe sulfinyle ou sulfo,

$R^1$ et $R^2$ désignent chacun, indépendamment l'un de l'autre, un atome d'hydrogène ou un radical alkyle en $C_1$ à $C_4$ et

$Z^1$, $Z^2$ et $Z^3$ représentent chacun, indépendamment des autres, un atome d'hydrogène ou d'halogène ou un groupe nitro, cyano, alkyle en $C_1$ à $C_4$, halo-alkyle en $C_1$ à $C_4$, alcoxy en $C_1$ à $C_4$, halo-alcoxy en $C_1$ à $C_4$, alkyl(en $C_1$ à $C_4$)-mercapto, halo-alkyl(en $C_1$ à $C_4$)-mercapto, alkyl(en $C_1$ à $C_4$)-sulfinyle ou alkyl(en $C_1$ à $C_4$)-sulfonyle,

n et p valent 0 ou1 et

m et q valent de 0 à 8, à l'exception de $m = q = 0$.

2. Diméthoxy-4,5 pyridazones substituées de la formule I suivant la revendication 1, caractérisées en ce que

X et Y désignent, indépendamment l'un de l'autre, un atome d'oxygène ou un atome de soufre,

$R^1$ et $R^2$ désignent, indépendamment l'un de l'autre, un atome d'hydrogène ou un radical alkyle en $C_1$ à $C_4$,

$Z^1$, $Z^2$ et $Z^3$ représentent chacun, indépendamment des autres, un atome d'hydrogène ou d'halogène ou un

radical alkyle en $C_1$ à $C_4$, halo-alkyle en $C_1$ à $C_4$, alcoxy en $C_1$ à $C_4$ ou halo-alcoxy en $C_1$ à $C_4$,
n et p valent 0 ou 1 et
m et q valent de 0 à 8, à l'exception de m = q = 0.

3. Diméthoxy-4,5 pyridazones substituées de la formule I suivant la revendication 1, caractérisées en ce que
$R^1 = R^2 = H$,
$Z^1 = H$,
$Z^2$ et $Z^3$ désignent un atome d'halogène,
n = 0,
m = 1,
p = 1 et
q = 0.

4. Procédé de préparation de diméthoxy-4,5 pyridazones substituées de la formule I suivant la revendication 1, caractérisé en ce que l'on fait réagir une dihalopyridazone de la formule IV

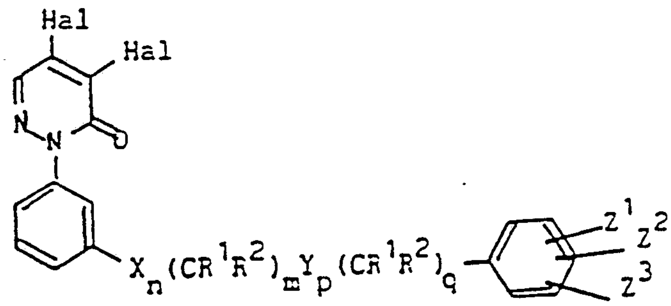

(IV),

dans laquelle X, Y, $R^1$, $R^2$, $Z^1$, $Z^2$, $Z^3$, n, m, p, et q possèdent les significations définies dans la revendication 1 et Hal désigne un atome d'halogène, dans un solvant organique inerte et à une température pouvant aller jusqu'à 100°C avec une proportion approximativement stoechiométrique d'un méthanolate d'un métal alcalin,

5. Procédé de préparation de diméthoxy-4,5 pyridazones substituées suivant la revendication 4, caractérisé en ce que l'on emploie une dihalo-pyridazone de la formule IV, obtenue par la réaction d'une phényl-hydrazine de la formule V

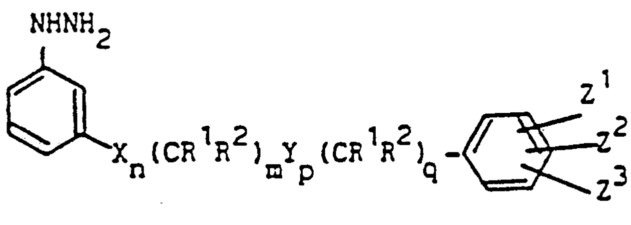

(V),

dans laquelle X, Y, $R^1$, $R^2$, $Z^1$, $Z^2$, $Z^3$, n, m, p et q possèdent les sigifications définies dans la revendication 1, avec un acide formyl-3 dihalo-2,3 acrylique de la formule VI

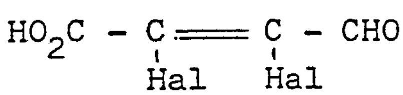

(VI),

dans laquelle Hal désigne un atome de chlore ou de brome, d'abord à la température normale en présence d'un solvant pour l'obtention de la semi-carbazone d'acide dihalo-acrylique correspondante, suivie de la cyclisation de cette semi-carbazone.

6. Procédé de préparation de diméthoxy-4,5 pyridazones substituées de la formule I suivant la revendication 1, dans laquelle X désigne un atome d'oxygène ou de soufre et n = 1, caractérisé en ce que l'on fait réagir une diméthoxy-4,5 pyridazone de la formule II

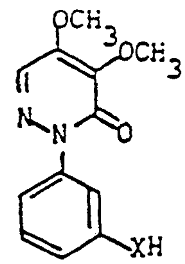

(II),

dans laquelle X désigne un atome d'oxygène ou de soufre, à une température comprise entre 0 et 150°C dans un solvant organique inerte et éventuellement en présence d'un fixateur d'acide, avec une proportion approximativement stoechiométrique d'un halogénure d'alkyle de la formule III

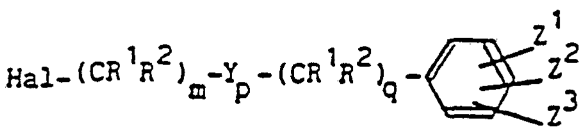

(III),

dans laquelle $R^1$, $R^2$, Y, $Z^1$, $Z^2$, $Z^3$, m, p et q possèdent les significations définies dans la revendication 1 et Hal désigne un atome d'halogéne.

7. Composition herbicide, contenant une diméthoxy-4,5 pyridazone de la formule I suivant la revendication 1.

8. Composition herbicide, contenant une diméthoxy-4,5 pyridazone de la formule I

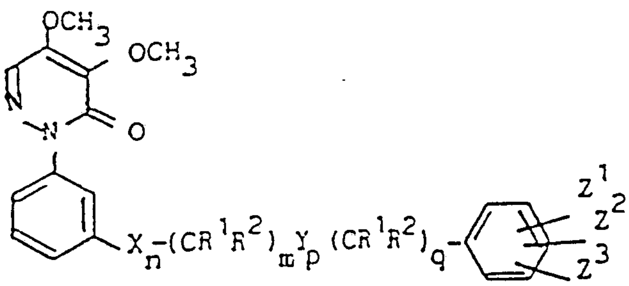

(I),

dans laquelle

X et Y peuvent désigner chacun, indépendamment l'un de l'autre, un atome d'oxygène ou de soufre ou un groupe sulfinyle ou sulfo,

$R^1$ et $R^2$ désignent chacun, indépendamment l'un de l'autre, un atome d'hydrogène ou un radical alkyle en $C_1$ à $C_4$ et

$Z^1$, $Z^2$ et $Z^3$ représentent chacun, indépendamment des autres, un atome d'hydrogène ou d'halogène ou un groupe nitro, cyano, alkyle en $C_1$ à $C_4$, haloalkyle en $C_1$ à $C_4$, alcoxy en $C_1$ à $C_4$, haloalcoxy en $C_1$ à $C_4$, alkyl(en $C_1$ à $C_4$)-mercapto, halo-alkyl(en $C_1$ à $C_4$)-mercapto, alkyl(en $C_1$ à $C_4$)-sulfinyle ou alkyl(en $C_1$à $C_4$)-sulfonyle,

n et p valent 0 ou1 et

m et q valent de 0 à 8, à l'exception de m=q=0, ainsi que des additifs inertes.

9. Composition herbicide, contenant une diméthoxy-4,5 pyridazone de la formule I suivant la revendication 1, dans laquelle

X et Y désignent, indépendamment l'un de l'autre, un atome d'oxygène ou un atome de soufre,

$R^1$ et $R^2$ désignent, indépendamment l'un de l'autre, un atome d'hydrogène ou un radical alkyle en $C_1$ à $C_4$,

$Z^1$, $Z^2$, $Z^3$ représentent chacun indépendamment des autres, un atome d'hydrogène ou d'halogène ou un radical alkyle en $C_1$ à $C_4$ , halo-alkyle en $C_1$ à $C_4$ , alcoxy en $C_1$ à $C_4$ ou halo-alcoxy en $C_1$ à $C_4$,

n et p valent 0 ou 1 et

m et q valent de 0 à 8, à l'exception de m=q=0, ainsi que des additifs inertes.

10. Procédé de lutte contre la croissance de plantes indésirables, caractérisé en ce que l'on traite les plantes ou les surfaces, sur lesquelles on souhaite empêcher le développement de plantes indésirables, avec une quantité efficace du point de vue herbicide d'une diméthoxy-4,5 pyridazone de la formule I suivant la revendication 1.